# EUROPÄISCHE PATENTSCHRIFT

(11) **EP 3 319 964 B1**
(45) Veröffentlichungstag und Bekanntmachung des Hinweises auf die Patenterteilung: **22.07.2020**
(21) Anmeldenummer: 16733295.6
(22) Anmeldetag: 20.06.2016
(51) Int. Cl.: C07D 487/04, C09D 17/00, C08K 5/3415, G02B 5/22

(54) **DITHIOALKYLPYRROLOPYRROLE UND IHRE VERWENDUNG ALS FARBSTOFFE**
DITHIO-ALKYL-PYRROLO-PYRROLES AND USE THEREOF AS DYES
DITHIOALKYLPYRROLOPYRROLES ET LEUR UTILISATION EN TANT QUE COLORANTS

(30) Priorität: 10.07.2015 EP 15002069
(43) Veröffentlichungstag der Anmeldung: 16.05.2018
(73) Patentinhaber: Merck Patent GmbH, 64293 Darmstadt (DE)
(72) Erfinder: KIRSCH, Peer, 64342 Seeheim-Jugenheim (DE); BECK, Susann, 64283 Darmstadt (DE); JUNGE, Michael, 64319 Pfungstadt (DE); PATWAL, Ursula, 64354 Reinheim (DE); FISCHER, Mila, 64367 Muehltal (DE)
(86) Internationale Anmeldenummer: PCT/EP2016/001048
(87) Internationale Veröffentlichungsnummer: WO 2017/008880

(56) Entgegenhaltungen:
- WO-A1-2009/141295
- WO-A1-2013/004677
- GANG QIAN ET AL: "Near-Infrared Thermochromic Diazapentalene Dyes", ADVANCED MATERIALS, Bd. 24, Nr. 12, 20. Februar 2012 (2012-02-20), Seiten 1582-1588, XP055295270, DE ISSN: 0935-9648, DOI: 10.1002/adma.201104711 in der Anmeldung erwähnt
- GANG QIAN ET AL: "Family of Diazapentalene Chromophores and Narrow-Band-Gap Polymers: Synthesis, Halochromism, Halofluorism, and Visible-Near Infrared Photodetectivity", CHEMISTRY OF MATERIALS, Bd. 24, Nr. 12, 26. Juni 2012 (2012-06-26) , Seiten 2364-2372, XP055295267, US ISSN: 0897-4756, DOI: 10.1021/cm300938s
- GANG QIAN ET AL: "Synthesis and study of low-bandgap polymers containing the diazapentalene and diketopyrrolopyrrole chromophores for potential use in solar cells and near-infrared photodetectors", JOURNAL OF MATERIALS CHEMISTRY, Bd. 22, Nr. 25, 1. Januar 2012 (2012-01-01), Seite 12867, XP055295265, GB ISSN: 0959-9428, DOI: 10.1039/c2jm30868a

## Beschreibung

### GEBIET DER ERFINDUNG

Die Erfindung befindet sich auf dem Gebiet der organischen Farbstoffchemie und betrifft die Verwendung dichroitischer Farbstoffe auf Basis von Dithioalkylpyrrolopyrrolen in flüssigkristallinen Mischungen.

### Stand der Technik

Auf dem Gebiet der Vorrichtungen zur Regulierung des Licht-Eintritts in einen Raum besteht Interesse an technischen Lösungen, mit denen haltbare und leistungsfähige Vorrichtungen erhalten werden können.

Ein vorteilhafter Ansatz für diese Vorrichtungen ist die Verwendung von Schaltschichten enthaltend eine Mischung aus mindestens einer flüssigkristallinen Verbindung in Kombination mit mindestens einem dichroitischen Farbstoff. Durch Anlegen einer Spannung kann bei diesen Schaltschichten eine Veränderung der räumlichen Ausrichtung der Farbstoffmoleküle erzielt werden, welche eine Änderung ihrer Absorption und damit der Transmission durch die Schaltschicht bewirkt. Eine entsprechende Vorrichtung ist beispielsweise in WO 2009/141295 beschrieben.

Zur Verwendung in den genannten Vorrichtungen sind bereits so genannte Rylen-Farbstoffe, speziell Perylen- und Terrylenderivate, beschrieben worden, beispielsweise in WO 2009 141295 A1 und WO 2013 004677 A1 (UNIV EINDHOVEN), deren Stabilität allerdings den extremen Anforderungen für die Anwendung in Fenstern nicht genügt.

Auch Benzothiadiazol- und Diketopyrroloprrolderivate sind als Bausteine für solche Farbstoffe bekannt. Allerdings eignen sich diese Verbindungen eher, den roten und gelben Farbraum abzudecken. Blaue und grüne Farbstoffe mit ausreichender Lichtechtheit und starkem Dichroismus basieren in der Regel auf Rylen-Strukturen, die bezüglich ihrer Löslichkeit in Flüssigkristallmischungen problematisch sind.

In diesem Zusammenhang sei auf folgende Druckschriften aus dem Stand der Technik hingewiesen:
Verbindungen mit Diketopyrrolopyrrol-Grundgerüst sind bereits seit längerem bekannt. In EP 0094911 A1 (CIBA) wird beschrieben, wie Diketopyrrolopyrrol-Verbindungen, die Arylgruppen als Substituenten aufweisen, effizient hergestellt werden können. Weiterhin ist die Verwendung dieser Verbindungsklasse als Fluoreszenzmarker und als Bestandteile von organischen Halbleitern und entsprechenden Halbleitervorrichtungen geläufig (WO 2004 090046 A1, BASF).

In dem Aufsatz von G. Qian und Z. Wang mit dem Titel "Near-IR thermochromic diazapentalene dyes", veröffentlicht in Adv. Mater. 24, S. 1582-1588 (2012**)** werden thermochrome Farbstoffe offenbart, die beispielsweise für unterschiedlichste Verwendungen in Betracht kommen, angefangen von Farben und Textilien bis hin zu Lichtfiltern und Biosensoren. Die Herstellung der Stoffe erfolgt ausgehend von Dithiophenpyrrolopyrroldionen, die zunächst mit Lawesson-Reagenz und dann einem Bromierungsmittel behandelt werden. Die dabei erhaltenen Dithiopyrrolopyrroldithione können dann mit einem Borsäurederivat in Gegenwart eines Palladiumkomplexes derivatisiert werden. Die Reaktion ist im nachfolgenden **Schema A** erläutert:

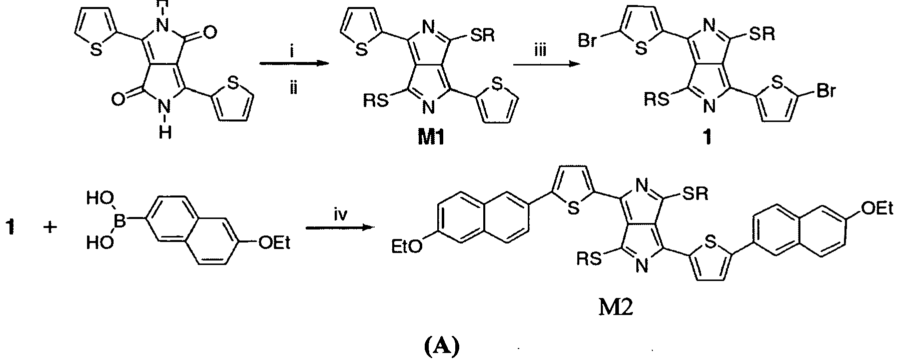

Konkret wird dabei lediglich die Substanz M2 offenbart, bei der die Derivatisierung durch Einführen eines Ethoxy-substituierten Naphthylrestes erfolgt. Die gleichen Stoffe werden von den gleichen Autoren auch in Chem. Mater. 24, S. 2364-2372 (2012**)** beschrieben.

Ebenfalls von den gleichen Autoren werden Dithiopyrrolopyrrol-Derivate beschrieben, die dem folgenden **Schema B** folgen:

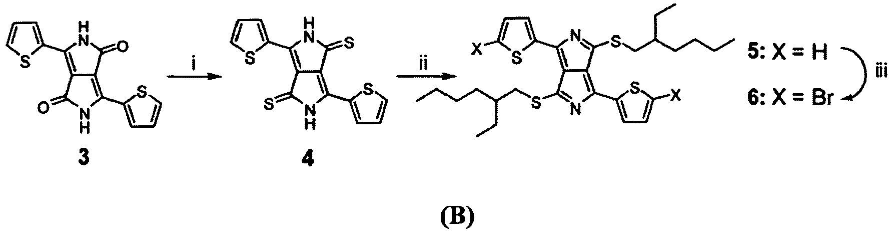

Die mit (5) und (6) bezeichneten Strukturen dienen dabei als Intermediate zur Herstellung von polymeren Chromophoren, wie im nachfolgenden **Schema C** angegeben:

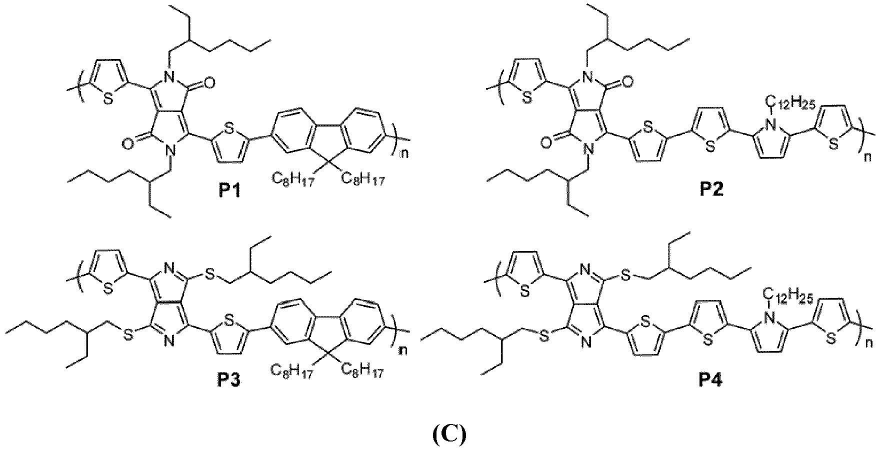

Bezüglich der dichroitischen Farbstoffe zur Verwendung in Vorrichtungen zur Regulierung des Lichteintritts in Räume besteht kontinuierlich Verbesserungsbedarf, insbesondere in Hinblick auf Lichtstabilität, Langzeitstabilität der Lösung und hohen Anisotropiegrad der Absorption.

Die Aufgabe der vorliegenden Erfindung hat daher darin bestanden, Farbstoffe mit dichroitischen Eigenschaften zur Verfügung zu stellen, die den Stoffen des Stands der Technik überlegen sind und sich insbesondere durch eine verbesserte Lichtechtheit und eine hohe Löslichkeit auszeichnen.

### Beschreibung der Erfindung

Ein erster Gegenstand der Erfindung betrifft die Verwendung mindestens eines Dithiopyrrolopyrrol-Derivats der Formel (1) 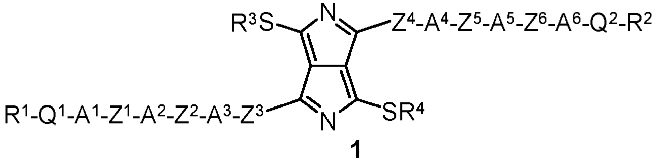 in einer flüssigkristallinen Mischung,
worin in der Formel (1)
- R¹, R², R³ und R⁴: unabhängig voneinander für lineare oder verzweigte, gegebenenfalls halogensubstituierte Alkylgruppen mit 1 bis 20 Kohlenstoffatomen oder Halogen stehen;
- Q¹ und Q²: jeweils Einfachbindungen bedeuten oder unabhängig voneinander für O, OCF₂, CF₂, CF₂CF₂, CO stehen;
- A¹,A², A³ und A⁴: jeweils Einfachbindungen bedeuten oder unabhängig voneinander für eine Gruppe stehen, die gebildet wird von Phenylen, Pyridin, Pyrimidin, Pyridazin, Naphthylen, Thiophen, Selenophen und Thiazol,
mit der Maßgabe, dass
(i) wenigsten A³ und A⁴ aromatische Ringstrukturen darstellen und
(ii) die aromatischen Ringstrukturen gegebenenfalls durch Halogenid substituiert sind; und
- Z¹, Z², Z³, Z⁴, Z⁵ und Z⁶: jeweils Einfachbindungen bedeuten oder unabhängig voneinander für ein Radikal stehen, das ausgewählt ist aus der Gruppe, die gebildet wird von C≡C, CH=CH, CF=CF, CF=CH, CH₂CH₂, CF₂-CF₂ und CF₂O,
mit der Maßgabe, dass
(iii) wenigstens Z³ und Z⁴ entweder Einfachbindungen oder eine π-konjugierte Gruppe darstellen.

Überraschenderweise wurde gefunden, dass die Stoffe der Formel (1) das Anforderungsprofil voll umfänglich erfüllen. Insbesondere zeigen die Stoffe in Lösung in einem flüssigkristallinen Host eine extreme Lichtechtheit, die speziell die von Rylen-Derivaten im Allgemeinen und Perylendiimid-Derivaten im Besonderen deutlich übersteigt. Gleichzeitig zeigen sie einen hohen Dichroismus sowie eine sehr gute Löslichkeit.

Im Rahmen der vorliegenden Erfindung werden unter einer Alkylgruppe mit 1 bis 20 C-Atomen bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Im Hinblick auf ihr Leistungsvermögen sind im Sinne der Erfindung solche Derivate der Formel (1) bevorzugt, bei denen
(i) R¹, R², R³ und R⁴ unabhängig voneinander für verzweigte Alkylreste mit 6 bis 12 Kohlenstoffatomen stehen; und/oder
(ii) 3, 4 oder 5 aromatische Ringstrukturen enthalten sind; und/oder
(iii) die aromatischen Ringstrukturen mit Fluorid substituiert sind.

Speziell bevorzugt sind Strukturen, die bei Temperaturen unterhalb von etwa 200 °C Mesophasen aufweisen. Zu diesen Stoffen zählen insbesondere die folgenden Verbindungen 2, 3, 4, 6 und 7: 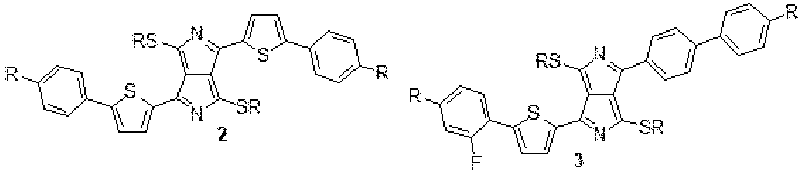 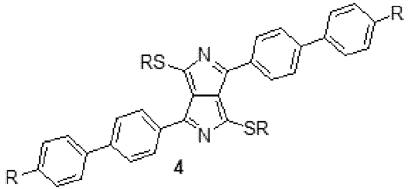 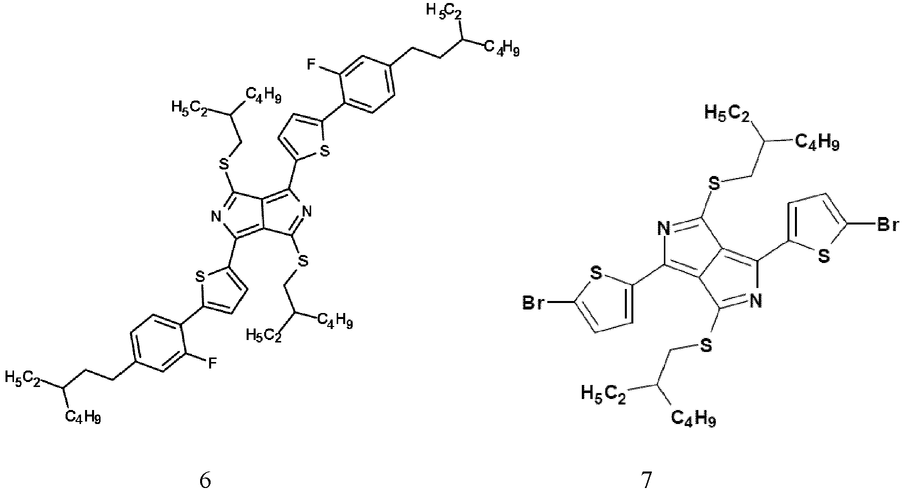 mit der Maßgabe, dass die Reste R die oben angegebene Bedeutung besitzen.

### HERSTELLVERFAHREN

Die Herstellung der gemäß Anspruch 1 verwendeten Dithiopyrrolopyrrol-Derivate kann allgemein durch das folgende **Schema D** beschrieben werden:

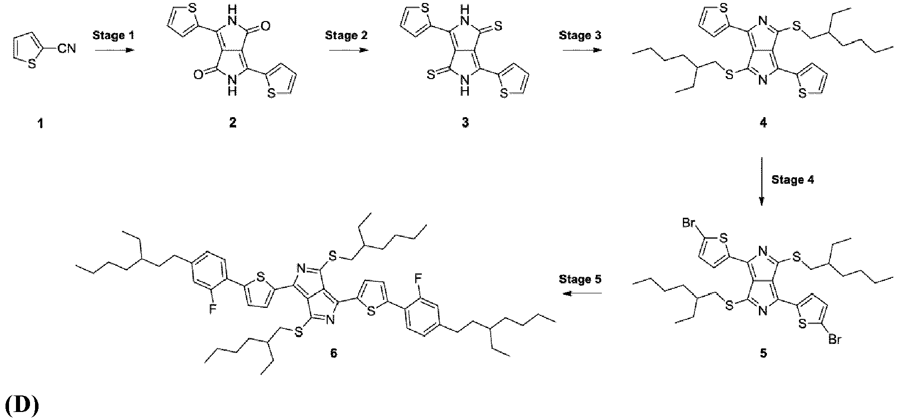

Die Synthesesequenz ist grundsätzlich aus dem Stand der Technik bekannt. Verwiesen wird auf den Artikel von F. Closs und R. Gompper mit dem Titel *"*2,5-Diazapentalen" in Angew. Chem. 99(6), S. 564-567 (1987**)** sowie die Aufsätze von G. Quian et al., die eingangs zitiert worden sind. Die Reaktionsschritte werden im Folgenden für ein konkretes Beispiel näher erläutert:

Der erste Schritt der Synthesesequenz kann durch Umsetzung von 2-Thiophencarbonitril mit Dimethylsuccinat in Gegenwart von Natriumhydrid und tert.-Amylalkohol als Lösungsmittel erfolgen.

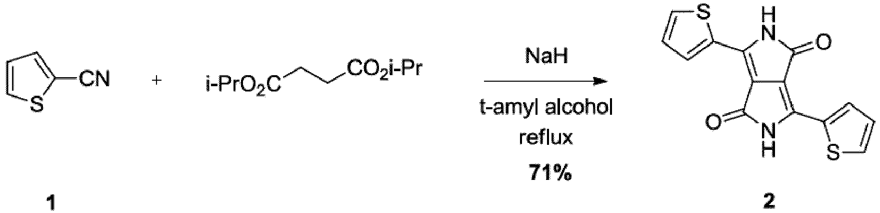

Vorzugsweise wird jedoch ein verzweigtes Substrat wie etwa Diisopropylsuccinat eingesetzt, da mit diesem höhere Ausbeute erzielt werden können.

Der zweite Schritt stellt die Thionierung dar, 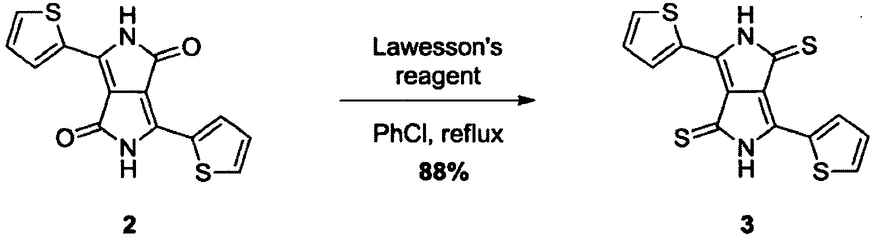 für die das aus dem Stand der Technik bekannte Laweson-Reagenz eingesetzt wird. Für die S-Alkylierung, die den dritten Schritt darstellt, wird ein entsprechendes Alkylbromid eingesetzt, 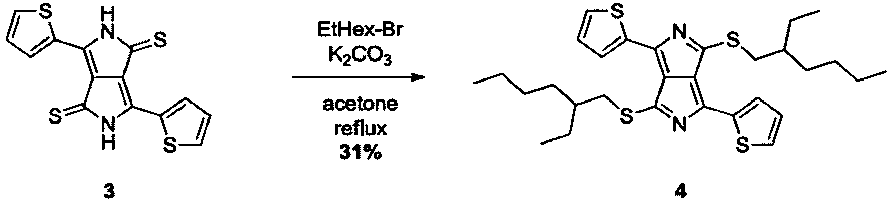 wobei als geeignetes Lösungsmittel beispielsweise Aceton in Frage kommt. Das Reaktionsprodukt kann beispielsweise mit Hilfe von Silikagel von überschüssigem Alkylierungsmittel befreit und dann aus Heptan umkristallisiert werden.

Es folgt die Bromierung, 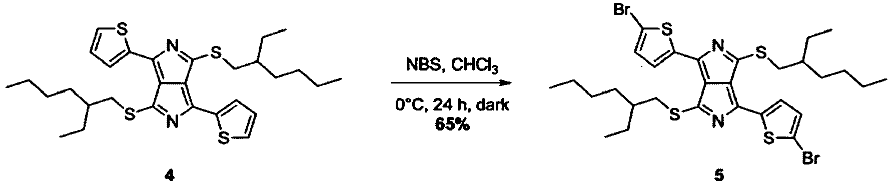 die beispielsweise durch Umsetzung mit N-Bromsuccinimid durchgeführt wird. Der letzte Verfahrensschritt stellt eine Suzuki-Kupplung dar, 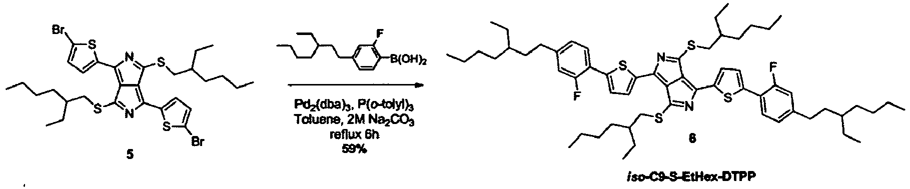 bei der die Gruppe, die das Bromid ersetzt, über eine Borsäureverbindung eingeführt wird. Als Katalysator kommen Übergangsmetallkomplexe vorzugsweise auf Basis von Palladium oder Platin in Frage, die als Liganden Phosphine oder Phosphite enthalten.

Für ein konkretes Beispiel kann der letzte Schritt auch durch das **Schema E** beschrieben werden:

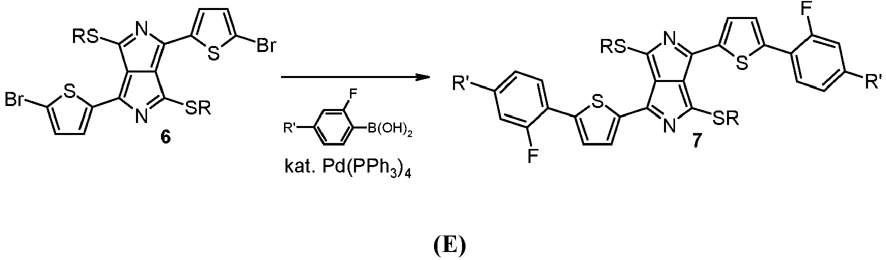

### MISCHUNGEN

Die Dithiopyrrolopyrrol-Derivate werden gemäß der vorliegenden Erfindung als Bestandteil von flüssigkristallinen Mischungen verwendet.

**Flüssigkristalline Verbindungen.** Die erfindungsgemäßen Mischungen enthalten insbesondere flüssigkristalline Verbindungen. Bevorzugt enthalten sie 3 bis 25 verschiedene flüssigkristalline Verbindungen, bevorzugt 8 bis 18, besonders bevorzugt 12 bis 16 verschiedene flüssigkristalline Verbindungen. Die flüssigkristallinen Verbindungen stellen bevorzugt die Hauptkomponenten der Mischung dar. Besonders bevorzugt sind sie zusammengenommen in einem Anteil von 90 bis 99.99 Gew.-%, besonders bevorzugt von 93 bis 99.9 Gew.-% und ganz besonders bevorzugt von 95 bis 99.8 Gew.-% in der Mischung vorhanden.

Die Verbindungen der Formel (1) liegen in den erfindungsgemäßen Mischungen bevorzugt in Lösung vor. Sie werden bevorzugt in ihrer Ausrichtung durch die Ausrichtung der flüssigkristallinen Verbindungen beeinflusst.

Die erfindungsgemäßen Mischungen stellen vorzugsweise flüssigkristalline Materialien dar. Weiterhin bevorzugt sind die Mischungen thermotrope flüssigkristalline Materialien, jedoch keine lyotropen flüssigkristallinen Materialien. Die erfindungsgemäßen Mischungen weisen bevorzugt einen Klärpunk auft, besonders bevorzugt einen Phasenübergang von einem nematisch flüssigkristallinen Zustand zu einem isotropen Zustand, im Temperaturbereich von 70 °C bis 170 °C, bevorzugt von 90 °C bis 160 °C, besonders bevorzugt von 95 °C bis 150 °C und ganz besonders bevorzugt von 105 °C bis 140 °C.

Bevorzugt ist weiterhin, dass die dielektrische Anisotropie der erfindungsgemäßen Mischungen größer als 3, besonders bevorzugt größer als 7 ist. Die dielektrische Anisotropie der erfindungsgemäßen Mischungen kann aber auch negativ sein. In diesem Fall haben sie bevorzugt einen Wert von -0.5 bis -10, besonders bevorzugt von -1 bis -8 und ganz besonders bevorzugt von -2 bis -6.

Weiterhin bevorzugt weisen die erfindungsgemäßen Mischungen eine optische Anisotropie (Δn) von 0.01 bis 0.3, besonders bevorzugt von 0.04 bis 0.27 auf. Flüssigkristalline Verbindungen, welche als Bestandteile der erfindungsgemäßen Mischung verwendet werden können, können gemäß dem allgemeinen Fachwissen des Fachmanns beliebig gewählt werden.

Es ist bevorzugt, dass die erfindungsgemäßen Mischungen als flüssigkristalline Verbindungen eine oder mehrere Verbindungen enthalten, die eine Nitrilgruppe aufweisen. Weiterhin ist es bevorzugt, dass die erfindungsgemäßen Mischungen als flüssigkristalline Verbindungen mindestens eine Verbindung enthalten, welche Strukturelemente basierend auf 1,4-Phenylenen und 1,4-Cyclohexylenen aufweisen. Besonders bevorzugt ist es, dass die erfindungsgemäßen Mischungen als flüssigkristalline Verbindung mindestens eine Verbindung aufweist, welche 2, 3 oder 4, besonders bevorzugt 3 oder 4 Strukturelemente basierend auf 1,4-Phenylenen und 1,4-Cyclohexylenen besitzen.

**Chirale Dotierstoffe.** Es ist weiterhin bevorzugt, dass die erfindungsgemäßen Mischungen einen oder mehrere chirale Dotierstoffe enthalten. Chirale Dotierstoffe werden in den erfindungsgemäßen Mischungen bevorzugt in einer Gesamtkonzentration von 0.01 bis 3 Gew.-%, besonders bevorzugt von 0.05 bis 1 Gew.-% verwendet. Um hohe Werte für die Verdrillung bei Verwendung der Mischung in einer Vorrichtung zu erhalten, kann die Gesamtkonzentration der chiralen Dotierstoffe auch höher als 3 Gew.-% gewählt werden, bevorzugt bis maximal 10 Gew.-%.

Gemäß einer alternativen, ebenfalls bevorzugten Ausführungsform, enthält die erfindungsgemäße Mischung keine chiralen Dotierstoffe.

**Stabilisatoren.** Die erfindungsgemäßen Mischungen enthalten weiterhin bevorzugt einen oder mehrere Stabilisatoren. Die Gesamtkonzentration der Stabilisatoren liegt bevorzugt zwischen 0.00001 und 10 Gew.-%, besonders bevorzugt zwischen 0.0001 und 1 Gew.-% der Mischung.

**Farbstoffverbindungen.** Die erfindungsgemäßen Mischungen enthalten bevorzugt zusätzlich zu der mindestens einen Verbindung der Formel (1) und der mindestens einen flüssigkristallinen Verbindung eine oder mehrere Farbstoffverbindungen mit anderer Struktur als Formel (1). Besonders bevorzugt enthält sie eine, zwei, drei oder vier Farbstoffverbindungen mit anderer Struktur als Formel (1), ganz besonders bevorzugt zwei oder drei Farbstoffverbindungen mit anderer Struktur als Formel (1). Diese Farbstoffverbindungen sind bevorzugt dichroitische Farbstoffverbindungen. Weiterhin sind es bevorzugt fluoreszierende Farbstoffverbindungen.

Bezüglich der Eigenschaft des Dichroismus gelten die für die Verbindung der Formel (1) beschriebenen bevorzugten Eigenschaften auch für die optionalen weiteren Farbstoffverbindungen mit anderer Struktur als Formel (1) als bevorzugt.

Die Absorptionsspektren der Farbstoffverbindungen der erfindungsgemäßen Mischungen ergänzen sich bevorzugt derart, dass für das Auge der Eindruck von schwarzer Farbe entsteht. Bevorzugt decken die Farbstoffverbindungen der erfindungsgemäßen Mischung einen großen Teil des sichtbaren Spektrums ab. Wie genau eine Mischung von Farbstoffverbindungen hergestellt werden kann, die für das Auge schwarz bzw. grau erscheint, ist dem Fachmann bekannt und beispielsweise in Manfred Richter, Einführung in die Farbmetrik, 2. Auflage, 1981, ISBN 3-11-008209-8, Verlag Walter de Gruyter & Co., beschrieben.

Die Einstellung des Farbortes einer Mischung aus Farbstoffverbindungen wird im Rahmen der Farbmetrik beschrieben. Hierzu werden die Spektren der Einzelfarbstoffe unter Berücksichtigung des Lambert-Beer'schen Gesetzes zu einem Gesamtspektrum berechnet und unter der zugehörigen Beleuchtung, z.B. für Tageslicht die Lichtart D65, gemäß den Regeln der Farbmetrik in die entsprechenden Farborte und Helligkeitswerte umgerechnet. Die Lage des Weißpunktes ist durch die jeweilige Lichtart, z.B. D65 festgelegt und in Tabellen (z.b. obenstehende Literaturstelle) aufgeführt. Durch Änderung der Anteile der verschiedenen Farbstoffverbindungen lassen sich verschiedene Farborte einstellen.

Der Anteil aller Farbstoffverbindungen in den erfindungsgemäßen Mischungen, unter Einschluss der mindestens einen Verbindung der Formel (1), beträgt bevorzugt insgesamt 0.01 bis 10 Gew.-%, besonders bevorzugt 0.1 bis 7 Gew.-% und ganz besonders bevorzugt 0.2 bis 5 Gew.-%.

Die Farbstoffverbindungen mit anderer Struktur als Formel (1) sind bevorzugt gewählt aus den in B. Bahadur, Liquid Crystals - Applications and Uses, Vol. 3, 1992, World Scientific Publishing, Abschnitt 11.2.1 angegebenen Farbstoffklassen und besonders bevorzugt aus den in der dort vorhandenen Tabelle aufgeführten expliziten Verbindungen.

Bevorzugt sind die Farbstoffverbindungen mit anderer Struktur als Formel (1) gewählt aus Azoverbindungen, Anthrachinonen, Methinverbindungen, Azomethinverbindungen, Merocyaninverbindungen, Naphthochinonen, Tetrazinen, Perylenen, Terrylenen, Quaterrylenen, höheren Rylenen, Benzothiadiazolen und Pyrromethenen. Besonders bevorzugt sind darunter Perylene, Terrylene, Benzothiadiazole und Azofarbstoffe.

Die genannten Farbstoffe gehören zu den dem Fachmann bekannten Klassen von dichroitischen Farbstoffen, die vielfach in der Literatur beschrieben sind. So sind z.B. Anthrachinonfarbstoffe beschrieben in EP 34832, EP 44893, EP 48583, EP 54217, EP 56492, EP 59036, GB 2065158, GB 2065695, GB 2081736, GB 2082196, GB 2094822, GB 2094825, JP-OS 55-123673, DE 3017877, DE 3040102, DE 3115147, DE 3115762, DE 3150803 und DE 3201120, Naphthochinonfarbstoffe beschrieben in DE 3126108 und DE 3202761, Azofarbstoffe in EP 43904, DE 3123519, WO 82/2054, GB 2079770, JP-OS 56-57850, JP-OS 56-104984, US 4308161, US 4308162, US 4340973, T. Uchida, C. Shishido, H. Seki und M. Wada: Mol. Cryst. Lig. Cryst. 39, 39-52 (1977) und H. Seki, C. Shishido, S. Yasui und T. Uchida: Jpn. J. Appl. Phys. 21, 191-192 (1982), und Rylene beschrieben in EP 2166040, US 2011/0042651, EP 68427, EP 47027, EP 60895, DE 3110960 und EP 698649.

### GEWERBLICHE ANWENDBARKEIT

Weitere Gegenstände der vorliegenden Erfindung betreffen die gewerbliche Anwendbarkeit der erfindungsgemäßen Farbstoffe, wie sie nachfolgend beschrieben wird:

### REGULIERUNG DES LICHTEINTRITTS

In einer ersten Ausgestaltungsform umfasst die Erfindung die Verwendung der erfindungsgemäßen Mischungen in einer Vorrichtung zur Regulierung des Lichteintritts in einen Raum.

Eine zweite Ausgestaltung ist in gleicher gedanklicher Verbindung auf eine Vorrichtung zur Regulierung des Lichteintritts in einen Raum gerichtet, welche die erfindungsgemäßen Mischungen enthält.

Die erfindungsgemäße Vorrichtung ist bevorzugt zur Regulierung des Licht-Eintritts in Form von der Sonne ausgehendem Licht von der Umgebung in einen Raum geeignet. Dabei erfolgt der zu regulierende Licht-Eintritt von der Umwelt (dem Außenraum) in einen Raum hinein. Der Raum kann dabei ein beliebiger weitgehend von der Umgebung abgeschlossener Raum sein, beispielsweise ein Gebäude, ein Fahrzeug, oder ein Container. Die Vorrichtung kann allgemein für beliebige Räume verwendet werden, besonders wenn diese nur begrenzten Luftaustausch mit der Umgebung aufweisen und lichtdurchlässige Begrenzungsflächen aufweisen, durch die Energie-Eintrag von außen in Form von Lichtenergie stattfinden kann. Besonders relevant ist die Verwendung der Vorrichtung für Räume, welche starker Sonneneinstrahlung durch lichtdurchlässige Flächen, beispielsweise durch Fensterflächen, ausgesetzt sind.

In einer alternativen Verwendung wird die Vorrichtung zur Regulierung des Lichteinfalls auf die Augen eingesetzt, beispielsweise in Schutzbrillen, Visieren oder Sonnenbrillen, wobei die Vorrichtung in einem Schaltzustand den Lichteinfall auf die Augen gering hält, und in einem anderen Schaltzustand den Lichteinfall weniger stark verringert.

Die erfindungsgemäße Vorrichtung ist bevorzugt in einer Öffnung einer größeren flächigen Struktur angeordnet, wobei die flächige Struktur selbst nicht oder nur geringfügig Licht-Eintritt zulässt, und wobei die Öffnung relativ gesehen lichtdurchlässiger ist. Bevorzugt ist die flächige Struktur eine Wand oder eine sonstige Begrenzung eines Raums nach außen. Weiterhin bevorzugt bedeckt die flächige Struktur eine mindestens genauso große Fläche, besonders bevorzugt eine mindestens doppelt so große Fläche wie die Öffnung in ihr, in der die erfindungsgemäße Vorrichtung angeordnet ist.

Bevorzugt ist die erfindungsgemäße Vorrichtung dadurch gekennzeichnet, dass sie eine Flächenausdehnung von mindestens 0.05 m² aufweist, bevorzugt mindestens 0.1 m², besonders bevorzugt mindestens 0.5 m² und ganz besonders bevorzugt mindestens 0.8 m².

Die erfindungsgemäße Vorrichtung ist schaltbar. Unter Schaltung wird dabei eine Änderung des Licht-Durchtritts durch die Vorrichtung verstanden. Die erfindungsgemäße Vorrichtung ist bevorzugt elektrisch schaltbar.

Ist die Vorrichtung elektrisch schaltbar, umfasst sie bevorzugt zwei oder mehr Elektroden, die zu beiden Seiten der Schaltschicht enthaltend die erfindungsgemäße Mischung angebracht sind. Die Elektroden bestehen bevorzugt aus ITO oder aus einer dünnen, bevorzugt transparenten Metall- und/oder Metalloxidschicht, beispielsweise aus Silber oder FTO (Fluor-dotiertes Zinnoxid) oder einem alternativen, dem Fachmann für diese Verwendung bekannten Material. Die Elektroden sind bevorzugt mit elektrischen Anschlüssen versehen. Die Spannung wird bevorzugt durch eine Batterie, einen Akkumulator, oder durch externe Stromversorgung bereitgestellt.

Der Schaltvorgang erfolgt im Fall von elektrischer Schaltung durch eine Ausrichtung der Moleküle der erfindungsgemäßen Mischung durch das Anlegen von Spannung.

In einer bevorzugten Ausführungsform wird dabei die Vorrichtung durch Anlegen einer Spannung von einem Zustand mit hoher Absorption, d. h. geringer Lichtdurchlässigkeit, der ohne Spannung vorliegt, in einen Zustand mit geringerer Absorption, d. h. höherer Lichtdurchlässigkeit, überführt. Bevorzugt ist die erfindungsgemäße Mischung in der Schicht in der Vorrichtung in beiden Zuständen nematisch. Der spannungslose Zustand ist bevorzugt dadurch gekennzeichnet, dass die Moleküle der Mischung, und damit die Moleküle der Verbindung der Formel (1), parallel zur Ebene der Schaltschicht ausgerichtet vorliegen. Dies wird bevorzugt durch eine entsprechend gewählte Orientierungsschicht erreicht. Der Zustand unter Spannung ist bevorzugt dadurch gekennzeichnet, dass die Moleküle der Mischung, und damit die Moleküle der Verbindung der Formel (1), senkrecht zur Ebene der Schaltschicht vorliegen.

In einer zur oben genannten Ausführungsform alternativen Ausführungsform wird die Vorrichtung durch Anlegen einer Spannung von einem Zustand mit geringer Absorption, d. h. hoher Lichtdurchlässigkeit, der ohne Spannung vorliegt, in einen Zustand mit höherer Absorption, d. h. geringerer Lichtdurchlässigkeit, überführt. Bevorzugt ist die erfindungsgemäße Mischung in der Schicht in der Vorrichtung in beiden Zuständen nematisch. Der spannungslose Zustand ist bevorzugt dadurch gekennzeichnet, dass die Moleküle der Mischung, und damit die Moleküle der Verbindung der Formel (1), senkrecht zur Ebene der Schaltschicht ausgerichtet vorliegen. Dies wird bevorzugt durch eine entsprechend gewählte Orientierungsschicht erreicht. Der Zustand unter Spannung ist bevorzugt dadurch gekennzeichnet, dass die Moleküle der Mischung, und damit die Moleküle der Verbindung der Formel (1), parallel zur Ebene der Schaltschicht vorliegen. Gemäß einer bevorzugten Ausführungsform der Erfindung kann die Vorrichtung ohne externe Stromversorgung betrieben werden, indem die nötige Energie durch eine Solarzelle oder eine sonstige Vorrichtung zur Umwandlung von Licht und/oder Wärmeenergie in elektrische Energie bereitgestellt wird, die mit der Vorrichtung verbunden ist. Die Bereitstellung der Energie durch die Solarzelle kann direkt oder indirekt, d. h. über eine dazwischengeschaltete Batterie oder Akkumulator oder sonstige Einheit zur Speicherung von Energie, erfolgen. Bevorzugt ist die Solarzelle außen an der Vorrichtung angebracht oder sie ist innerer Bestandteil der Vorrichtung, wie beispielsweise in WO 2009 141295 A1 offenbart. Bevorzugt sind dabei insbesondere Solarzellen, welche bei diffusem Licht besonders effizient sind, sowie transparente Solarzellen.

Eine solche Vorrichtung kann sich dadurch auszeichnen, dass sie folgende Schichtabfolge aufweist, wobei zusätzlich weitere Schichten vorhanden sein können:
- Substratschicht;
- Elektrisch leitfähige transparente Schicht;
- Orientierungsschicht;
- Schaltschicht enthaltend die Dithiopyrrolopyrrol-Derivate;
- Orientierungsschicht;
- Elektrisch leitfähige transparente Schicht;
- Substratschicht.

Die bevorzugten Ausführungsformen der einzelnen Schichten werden im Folgenden dargestellt.

Bevorzugt umfasst die erfindungsgemäße Vorrichtung eine oder mehrere, besonders bevorzugt zwei Orientierungsschichten. Die Orientierungsschichten liegen bevorzugt direkt angrenzend an die beiden Seiten der Schicht enthaltend die erfindungsgemäße Mischung vor.

Als Orientierungsschichten der erfindungsgemäßen Vorrichtung können beliebige dem Fachmann hierzu bekannte Schichten verwendet werden. Bevorzugt sind Polyimid-Schichten, besonders bevorzugt Schichten aus geriebenem Polyimid. Auf bestimmte, dem Fachmann bekannte Art geriebenes Polyimid führt zu einer Ausrichtung der Moleküle des flüssigkristallinen Mediums in Reiberichtung, wenn die Moleküle parallel zur Orientierungsschicht vorliegen (planare Ausrichtung). Dabei ist es bevorzugt, dass die Moleküle des flüssigkristallinen Mediums nicht völlig planar auf der Orientierungsschicht vorliegen, sondern einen leichten Aufstellwinkel aufweisen (pretilt). Zum Erreichen von vertikaler Ausrichtung der Verbindungen des flüssigkristallinen Mediums zur Oberfläche der Orientierungsschicht (homeotrope Ausrichtung) wird bevorzugt auf bestimmte Art behandeltes Polyimid als Material für die Orientierungsschicht eingesetzt (Polyimid für sehr hohe Pretiltwinkel). Weiterhin können Polymere, die durch einen Belichtungsvorgang mit polarisiertem Licht erhalten wurden, als Orientierungsschicht zum Erreichen einer Ausrichtung der Verbindungen des flüssigkristallinen Mediums gemäß einer Orientierungsachse verwendet werden (photoalignment).

Weiterhin bevorzugt ist in der erfindungsgemäßen Vorrichtung die Schicht enthaltend die erfindungsgemäße Mischung zwischen zwei Substratschichten angeordnet bzw. von diesen eingeschlossen. Die Substratschichten können beispielsweise aus Glas oder einem Polymer, bevorzugt einem lichtdurchlässigen Polymer, bestehen.

Bevorzugt ist die Vorrichtung dadurch gekennzeichnet, dass sie keinen Polarisator auf Polymerbasis, besonders bevorzugt keinen in fester Materiephase vorliegenden Polarisator und ganz besonders bevorzugt überhaupt keinen Polarisator umfasst.

Die Vorrichtung kann jedoch gemäß einer alternativen Ausführungsform auch einen oder mehrere Polarisatoren aufweisen. Bevorzugt sind die Polarisatoren in diesem Fall Linearpolarisatoren.

Wenn genau ein Polarisator vorhanden ist, so ist dessen Absorptionsrichtung bevorzugt senkrecht stehend zur Orientierungsachse der Verbindungen des flüssigkristallinen Mediums der erfindungsgemäßen Vorrichtung auf derjenigen Seite der Schaltschicht, auf der sich der Polarisator befindet.

In der erfindungsgemäßen Vorrichtung können sowohl absorptive als auch reflektive Polarisatoren eingesetzt werden. Bevorzugt werden Polarisatoren verwendet, die als dünne optische Filme vorliegen. Beispiele für reflektive Polarisatoren, die in der erfindungsgemäßen Vorrichtung verwendet werden können, sind DRPF- (diffusive reflective polariser film, 3M), DBEF- (dual brightness enhanced film, 3M), DBR-(layered-polymer distributed Bragg reflectors, wie in US 7,038,745 und US 6,099,758 beschrieben) und APF-Filme (advanced polariser film, 3M, vgl. Technical Digest SID 2006, 45.1, US 2011/0043732 und US 7023602). Weiterhin können Polarisatoren basierend auf Drahtgittern (WGP, wire-grid-polarisers), welche Infrarotlicht reflektieren, eingesetzt werden. Beispiele für absorptive Polarisatoren, welche in den erfindungsgemäßen Vorrichtungen eingesetzt werden können, sind der Itos XP38-Polarisatorfilm und der Nitto Denko GU-1220DUN-Polarisatorfilm. Ein Beispiel für einen circularen Polarisator, welcher erfindungsgemäß verwendet werden kann, ist der Polarisator APNCP37-035-STD (American Polarizers). Ein weiteres Beispiel ist der Polarisator CP42 (ITOS).

Weiterhin bevorzugt enthält die erfindungsgemäße Vorrichtung ein Lichtleitsystem, das Licht zu einer Solarzelle oder einer sonstigen Vorrichtung zur Umwandlung von Licht und/oder Wärmeenergie in elektrische Energie leitet, bevorzugt wie in WO 2009/141295 beschrieben. Das Lichtleitsystem sammelt und konzentriert Licht, das auf die Vorrichtung trifft. Bevorzugt sammelt und konzentriert es Licht, das von fluoreszierenden dichroitischen Farbstoffen in der Schaltschicht emittiert wird. Das Lichtleitsystem steht mit einer Vorrichtung zur Umwandlung von Lichtenergie in elektrische Energie, bevorzugt einer Solarzelle, in Kontakt, so dass das gesammelte Licht konzentriert auf diese trifft. In einer bevorzugten Ausführungsform der Erfindung ist die Vorrichtung zur Umwandlung von Lichtenergie in elektrische Energie am Rand der Vorrichtung angebracht, in diese integriert und elektrisch mit Mitteln zur elektrischen Schaltung der erfindungsgemäßen Vorrichtung verbunden.

In einer bevorzugten Ausführungsform ist die erfindungsgemäße Vorrichtung Bestandteil eines Fensters, besonders bevorzugt eines Fensters enthaltend mindestens eine Glasfläche, ganz besonders bevorzugt eines Fensters, welche Mehrscheiben-Isolierglas enthält.

Unter Fenster wird dabei insbesondere eine Struktur in einem Gebäude verstanden, welche einen Rahmen und mindestens eine von diesem Rahmen umfasste Glasscheibe enthält. Bevorzugt enthält sie einen wärmeisolierenden Rahmen und zwei oder mehr Glasscheiben (Mehrscheiben-Isolierglas).

Gemäß einer bevorzugten Ausführungsform ist die erfindungsgemäße Vorrichtung direkt auf einer Glasfläche eines Fensters aufgebracht, besonders bevorzugt im Zwischenraum zwischen zwei Glasscheiben von Mehrscheiben-Isolierglas.

Schließlich wird in gleicher gedanklicher Verbindung auch ein Fenster beansprucht, welches eine wie oben erläuterte Vorrichtung enthält oder aufweist.

Für die vorliegende Erfindung und in den folgenden Beispielen sind die Strukturen der Flüssigkristallverbindungen durch Acronyme angegeben, wobei die Transformation in chemische Formeln gemäß folgender Tabellen A bis C erfolgt. Alle Reste CₙH₂ₙ₊₁, CₘH₂ₘ₊₁ und CₗH₂ₗ₊₁ bzw. CₙH₂ₙ, CₘH₂ₘ und CₗH₂ₗ sind geradkettige Alkylreste bzw. Alkylenreste jeweils mit n, m bzw. l C-Atomen. In Tabelle A sind die Ringelemente der Kerne der Verbindung codiert, in Tabelle B sind die Brückenglieder aufgelistet und in Tabelle C sind die Bedeutungen der Symbole für die linken bzw. rechten Endgruppen der Moleküle aufgelistet. Die Akronyme werden aus den Codes für die Ringelemente mit optionalen Verknüpfungsgruppen, gefolgt von einem ersten Bindestrich und den Codes für die linke Endgruppe, sowie einem zweiten Bindestrich und den Codes für die rechts Endgruppe, zusammengesetzt.

**Tabelle A: Ringelemente**

| | | | |
|---|---|---|---|
| **C** | 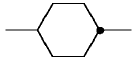 | | |
| **D** | 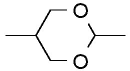 | **DI** | 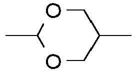 |
| **A** | 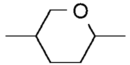 | **AI** | 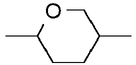 |
| **P** | 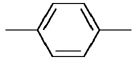 | | |
| **G** | 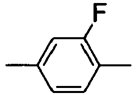 | **GI** | 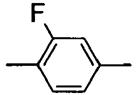 |
| **U** | 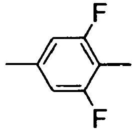 | **UI** | 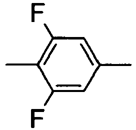 |
| **Y** | 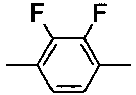 | | |
| **P(F,Cl)** | 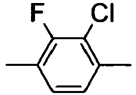 | **P(Cl,F)** | 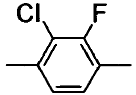 |
| **N** | 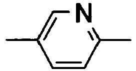 | **NI** | 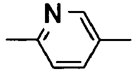 |
| **M** | 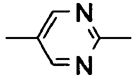 | **MI** | 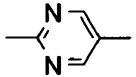 |
| **np** | 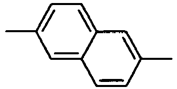 | | |
| **n3f** | 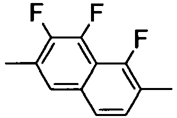 | **n3fI** | 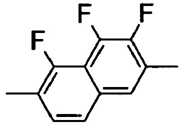 |
| **th** | 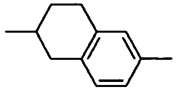 | **thI** | 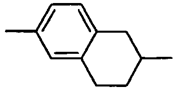 |
| **tH2f** | 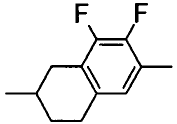 | **tH2fI** | 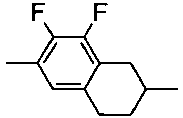 |
| **o2f** | 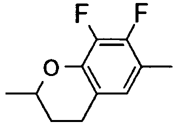 | **o2fI** | 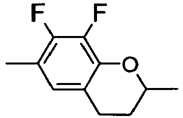 |
| **dh** | 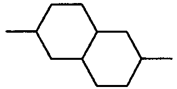 | | |
| **K** | 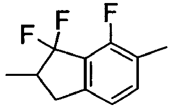 | **KI** | 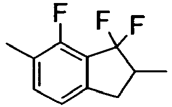 |
| **L** | 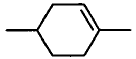 | **LI** | 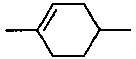 |
| **F** | 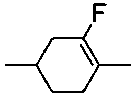 | **FI** | 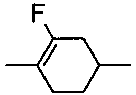 |

**Tabelle B: Brückenglieder**

| | | | |
|---|---|---|---|
| **E** | -CH₂-CH₂- | | |
| **V** | -CH=CH- | | |
| **T** | -C≡C- | | |
| **W** | -CF₂-CF₂- | | |
| **B** | -CF=CF- | | |
| **Z** | -CO-O- | **ZI** | -O-CO- |
| **X** | -CF=CH- | **XI** | -CH=CF- |
| **O** | -CH₂-O- | **OI** | -O-CH₂- |
| **Q** | -CF₂-O- | **QI** | -O-CF₂- |

**Tabelle C: Endgruppen**

| **Links einzelst tehend oder in Kombination** | | **Rechts einz elstehend oder in Kombination** | |
|---|---|---|---|
| **-n-** | CₙH₂ₙ₊₁- | **-n** | -CₙH₂ₙ₊₁ |
| **-nO-** | CₙH₂ₙ₊₁-O- | **-nO** | -O- CₙH₂ₙ₊₁ |
| **-V-** | CH₂=CH- | **-V** | -CH=CH₂ |
| **-nV-** | CₙH₂ₙ₊₁-CH=CH- | **-nV** | -CₙH₂ₙ-CH=CH₂ |
| **-Vn-** | CH₂=CH- CₙH₂ₙ- | **-Vn** | -CH=CH-CₙH₂ₙ₊₁ |
| **-nVm-** | CₙH₂ₙ₊₁-CH=CH-CₘH₂ₘ- | **-nVm** | - CₙH₂ₙ-CH=CH-CₘH₂ₘ₊₁ |
| **-N-** | N≡C- | **-N** | -C≡N |
| **-S-** | S=C=N- | **-S** | -N=C=S |
| **-F-** | F- | **-F** | -F |
| **-CL-** | Cl- | **-CL** | -Cl |
| **-M-** | CFH₂- | **-M** | -CFH₂ |
| **-D-** | CF₂H- | **-D** | -CF₂H |
| **-T-** | CF₃- | **-T** | -CF₃ |
| **-MO-** | CFH₂O - | **-OM** | -OCFH₂ |
| **-DO-** | CF₂HO- | **-OD** | -OCF₂H |
| **-TO-** | CF₃O - | **-OT** | -OCF₃ |
| **-A-** | H-C≡C- | **-A** | -C≡C-H |
| **-nA-** | CₙH₂ₙ₊₁-C≡C- | **-An** | -C≡C-CₙH₂ₙ₊₁ |
| **-NA-** | N≡C-C≡C- | **-AN** | -C≡C-C≡N |

| **Links nur in Kombination** | | **Rechts nur in Kombination** | |
|---|---|---|---|
| **-...n...-** | -CₙH₂ₙ- | **-...n...** | -CₙH₂ₙ- |
| **-...M...-** | -CFH- | **-...M...** | -CFH- |
| **-...D...-** | -CF₂- | **-...D...** | -CF₂- |
| **-...V...-** | -CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

worin n und m jeweils ganze Zahlen und die drei Punkte "..." Platzhalter für andere Abkürzungen aus dieser Tabelle sind.

### BEISPIELE

### REFERENZBEISPIEL H1

### Herstellung von 3,6-di(thiophen-2-yl)pyrrolo[3,4-c]pyrrol-1,4(2H,5H)-dion (DKPP) (2)

Die Herstellung der Vorstufe (2) erfolgt gemäß folgendem Reaktionsschema (I):

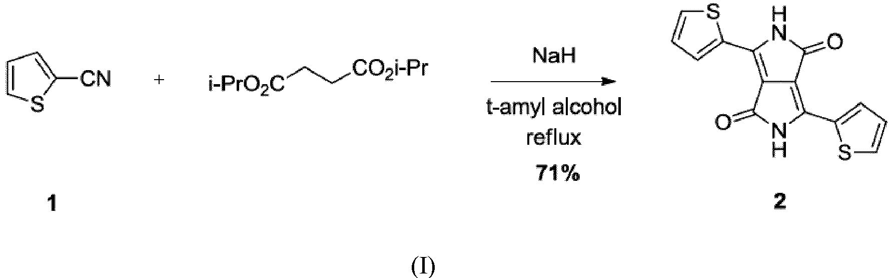

24,5 g (0,610 mol) einer 60 %-igen Natriumhydrid-Suspension wird unter Stickstoff in etwa 10 Portionen zu 670 g 2-Methyl-2-butanol gegeben und dem dabei auftretenden Schaum Gelegenheit gegeben, zusammenzufallen. Die trübe Lösung wird anschließend auf 60 °C erwärmt, wobei eine gelbe Lösung entsteht, zu der in einer Portion 66,7 g (0,610 mol) 2-Thiophencarbonitril (1) zugegeben wird; dabei wird eine bräunlich gefärbte Dispersion erhalten. Die Temperatur wird auf 100 °C erhöht, wobei die Suspension sich klärt und eine braune Lösung erhalten wird. Über einen Zeitraum von einer Stunde wird die Lösung tropfenweise mit 49,4 g (0,244 mol) Diisopropylsuccinat versetzt, wobei die Lösung vorsichtig bis zum Rückfluss erhitzt wird. Die dabei resultierende violette Lösung wird dann für weitere 14 Stunden unter Rückfluss gehalten und dann auf Raumtemperatur abgekühlt. Der Reaktionsansatz wird nachfolgend langsam in 3 L Methanol eingerührt und mit 160 ml Eisessig versetzt, 2 weitere Stunden gerührt und der ausgefallene Feststoff abfiltriert. Der Filterkuchen wird mit 3 L Wasser gewaschen, bis das Filtrat farblos ist und ein weiteres Mal mit 1 L O.P. IMS gespült. Anschließend wird der Filterkuchen über Nacht bei 80 °C im Hochvakuum getrocknet, wobei 52 g DKPP **(2)** entsprechend einer Ausbeute von 71 % der Theorie erhalten werden.

HPLC-Auswertung: 99,8 % (280 nm), 99,7 % (530 nm);
Elementaranalyse (C₁₄H₈N₂O₂S₂): theoretisch C: 55,99 %; H: 2,68 %; N: 9,33 % - gefunden C: 55,73 %; H: 2,61 %; N: 9,17 %).

Das Produkt kann als analytisch rein klassifiziert werden.

### REFERENZBEISPIEL H2

### Herstellung 3,6-di(thiophen-2-yl)pyrrolo[3,4-c]pyrrol-1,4(2H,5H)-dithion (DTPP) (3)

Die Herstellung der Vorstufe **(3)** erfolgt gemäß folgendem Reaktionsschema (II):

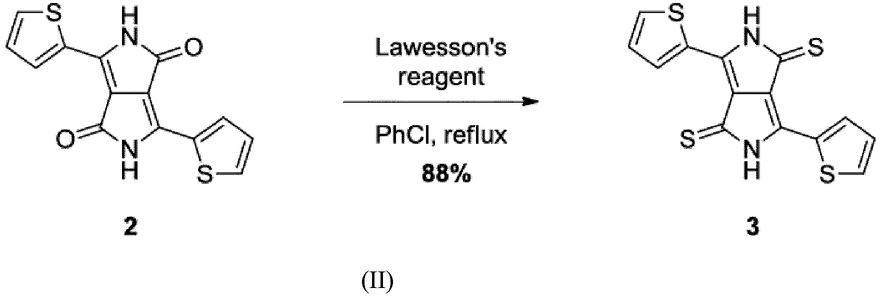

51,0 g (0,17 Mol) der Vorstufe (2) aus Herstellbeispiel H1 und 137 g (0,34 Mol) Lawesson's Reagenz werden unter Stickstoff in einen 3 L-Kolben gegeben und mit 2 L Chlorbenzol versetzt. Die Mischung wird unter Inertgas für 12 Stunden unter Rückfluss erhitzt, wobei der suspendierte Feststoff die Farbe von Rot über Purpur nach Schwarz ändert. Die Mischung wird auf Raumtemperatur abgekühlt, filtriert und der Filterkuchen mit 500 ml Methanol gewaschen. Anschließend wird der Rückstand in 2 L Methanol resuspendiert und eine Stunde bei 45 bis 50 °C gerührt und im heißen Zustand filtriert. Der Filterkuchen wird zunächst mit 1 L Methanol und dann mit 1 L Aceton gewaschen und über Nacht in einem Exsikkator getrocknet. Es werden 55,0 g DTPP **(3)** in Form eines schwarzen Pulvers erhalten, was einer Ausbeute von 97 % der Theorie entspricht.

HPLC-Auswertung: 94,8 % (330 nm); 97,5 % (625 nm)
Elementaranalyse (C₁₄H₈N₂O₂S₄): theoretisch N: 8,43 % - gefunden N: 7,57 %).

### REFERENZBEISPIEL H3

### Herstellung von 3,6-di(thiophen-2-yl)pyrrolo[3,4-c]pyrrol-[Di-S-EtHex-DTTP] (4)

Die Herstellung der Vorstufe **(4)** erfolgt gemäß folgendem Reaktionsschema (III):

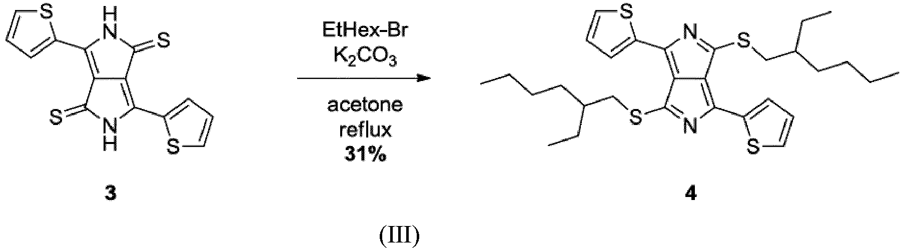

53,2 g (0,144 Mol) der Vorstufe **(3)** aus Herstellbeispiel H2, 88,3 g (0,64 Mol) Kaliumcarbonat und 92,6 g (0,48 Mol) 2-Ethylhexylbromid werden unter Stickstoff in einen 5 L Kolben gegeben, mit 3,2 L Aceton versetzt und 24 h unter Rückfluss gehalten. Nach Abkühlen auf Raumtemperatur werden anorganische Salze abgetrennt und das Filtrat bis zur Trockne eingedampft, wobei ein schwarzer Feststoff erhalten wird. Dieser wird in 200 ml Dichlormethan gelöst und in der Wärme mit 1 L Heptan versetzt. Am Rotationsverdampfer wird das Dichlormethan bei 70 °C abdestilliert. Die zurückbleibende Lösung wird auf ein Silicagel-Bett (40-63 µ, 1 kg, 19 cm Ø) gegeben, das mit Heptan angefeuchtet ist. Das Bett wird mit einer 3:1 Mischung aus Heptan und Dichlormethan eluiert, wobei 5 x 3 Fraktionen gewonnen werden; der Vorgang wird abgebrochen, wenn das Eluat beginnt sich zu verfärben. Die reinsten Fraktionen 2 und 3 werden vereinigt und bis zur Trockne eingedampft, wobei 48,3 g eines schwarzen Rückstands erhalten werden, entsprechend 54 % der Theorie. Der Rückstand wird in 100 ml Dichlormethan resuspendiert und bei 40 °C mit weiteren 500 ml Heptan versetzt. Anschließend wird das Dichlormethan wieder am Rotationsverdampfer bei 70 °C abgetrennt und die zurückbleibende Lösung in 50 ml heißes Heptan filtriert. Das Heptan wird abdestilliert, wobei 500 ml Lösung zurückbleibt, die über Nacht stehen gelassen wird. Die in dieser Zeit ausgefallenen Kristalle werden abfiltriert und auf dem Filter zunächst mit zwei Portionen a 30 ml kaltem Heptan (-20 °C) und dann mit zwei Portionen a 30 ml Acetonitril gewaschen. Nach dem Trocknen wird die Vorstufe **(4)** als schwarz-violette Kristalle in einer Menge von 28,0 g entsprechend 31 % der Theorie erhalten.

HPLC-Auswertung: 99,3 % (330 nm); 100 % (550 nm); 98,5 % (625 nm)
¹H-NMR: (CDCl₃, 500 MHz) δ 0.85 (6H, t, J 7.0), 0,92 (6H, t, J7.5), 1,20-1,55 (16H, m), 1,76 (2H, m), 3,47 (4H, m), 7,20 (2H, m), 7,56 (2H, br. S), 8,03 (2H br. s).

### REFERENZBEISPIEL H4

### Herstellung von 3,6-di(thiophen-2-yl)pyrrolo[3,4-c]pyrrol-[Di-Br-di-S-EtHex-DTTP] (5)

Die Herstellung der Vorstufe **(5)** erfolgt gemäß folgendem Reaktionsschema (IV):

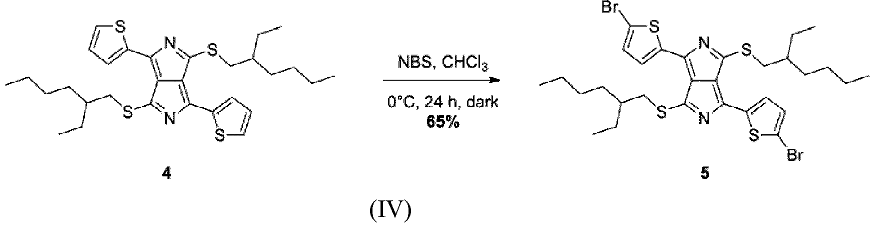

27,9 g (50 mMol) der Vorstufe **(4)** aus Herstellbeispiel H3 werden in 1,4 L Chloroform gelöst und im Eisbad auf -5°C abgekühlt. Der Kolben wird mit Aluminiumfolie abgedeckt, um Licht auszuschließen und die Mischung dann über einen Zeitraum von einer Stunde in 10 Portionen mit insgesamt 19,1 g (107 mMol) N-Bromsuccinimid versetzt. Anschließend wird die Mischung über 48 Stunden bei Umgebungstemperatur gerührt. Anschließend wird sie im Vakuum auf die Hälfte ihres Volumens eingeengt, wobei sich ein Feststoff abscheidet. Dieser wird durch Erhitzen auf 60 °C wieder in Lösung gebracht und dann mit 700 ml Methanol versetzt, wodurch er erneut ausgefällt wird. Daraufhin wird die Mischung wieder auf die Hälfte ihres Volumens eingeengt, mit weiteren 700 ml Methanol versetzt, rasch für 5 Minuten auf 60 °C erwärmt und heiß filtriert. Der Rückstand wird auf dem Filter mit 4 Portionen a 300 ml Methanol gewaschen, bis das ursprünglich bräunliche Filtrat farblos wird. Nach dem Trocknen wird die Vorstufe **(5)** als schwarzes Pulver in einer Menge von 23,4 g entsprechend 65 % der Theorie erhalten.

HPLC-Auswertung: 99,4 % (330 nm); 100 % (550 nm); 99,7 % (625 nm)
¹H-NMR: (CDCl₃, 500 MHz) δ 0,94 (6H, t, J 7,0), 0,98 (6H, t, J 7,5), 1H, m), 3,45 (2H, dd, J 6,5, J 13,5), 3,54 (2H, dd, J 6,0, J 13,5), 7,22 (2H, d, J 4,0), 7,81, d, J 4.0).

### REFERENZBEISPIEL H5

### Herstellung von 1,4-bis-(5-(4-(3-ethylheptyl)-2-fluorophenyl)thiophen-2-yl-3,6-bis((2-ethylhexyl)thio)pyrrolo[3,4-c]-pyrrol (6)

Die Herstellung des erfindungsgemäßen Produktes **(6)** erfolgt gemäß folgendem Reaktionsschema (V):

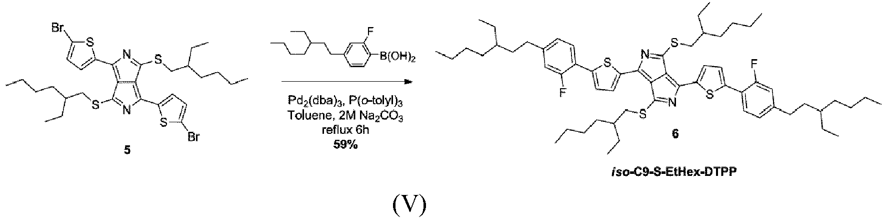

1,63 g (6,12 mmol) (4-3-Ethylheptyl)-2-fluorophenyl)borsäure und 1,90 g (2,66 mMol) der Vorstufe **(5)** aus Herstellbeispiel H4 werden in einem Kolben vorgelegt, mit 70 ml Toluol und 12 ml Wasser versetzt und stark gerührt. Die Mischung wird entlüftet und dreimal unter Stickstoff gesetzt, ehe 2,54 g (24,9 mMol) Natriumcarbonat, 0,155 g (0,377 mMol) Tris(o-tolyl)phosphin und 0,086 g (0,094 mMol) Tris(dibenzyliden)dipalladium hinzugegeben werden. Der Kolben wird mit einem Rückflusskühler versehen und die Mischung unter Stickstoff über einen Zeitraum von 6 Stunden am Rückfluss gehalten, ehe sie über Nacht abkühlt. Nach Filtration wird die Lösung in einen Scheidetrichter überführt und die untere wässrige Phase abgetrennt und verworfen. Die organische Phase wird mit Natriumsulfat getrocknet und dann bis zur Trockne eingeengt, wobei eine grün-schwarze ölige, halbfeste Masse erhalten wird.

Dieser Rückstand wird in einer minimalen Menge einer 50:50 Mischung Dichlormethan/Heptan aufgenommen und über eine Silikakolonne gegeben (40-63 µ, 200 g, 5 cm Ø), welche mit dem gleichen Lösungsmittel gepackt ist. Die Kolonne wird mit dem Laufmittel eluiert und 50 ml-Fraktionen abgenommen, bis sich das Eluat grün zu färben beginnt. Die produktreichen Fraktionen 2 bis 4 werden vereinigt und vom Lösungsmittel befreit, wobei 2,1 g eines schwarzen Feststoffs anfallen, entsprechend 79 % der Theorie. Der Feststoff wird aus 50 ml 2-Butanon umkristallisiert, der Rückstand abfiltriert und auf dem Filter mit HPLC-reinen Aceton gewaschen bevor er im Trockenschrank bei 40 °C von restlichem Lösungsmittel befreit wird. Der erfindungsgemäße Farbstoff **(6)** wird als bronzefarbige Mikrokristalle in einer Menge von 1,56 g, entsprechend 59 % der Theorie erhalten.

HPLC-Auswertung: 99,0 % (280 nm); 99,5 % (370 nm); 99,4 % (470 nm), 99,5 % (600 nm), 99,4 % (650 nm)
UV-vis (CDCl₃) λₘₐₓ 656 nm (61.000 FWHM 105 nm), 609 (46.500) 473 (30.000, FWHM 48 nm), 370 (28.500)
¹H-NMR: (CDCl₃, 500 MHz) δ 0,86-0,95 (18 H, m), 1,01 (6H, t, J 7,5), 1,20-1,65 (38 H, m), 1,85 (2H, m), 2,61 (4H, m), 3,51 (2H, dd, J 6,5 J 13,0), 3,60 (2H, dd, J 6,0, J13,0), 6,97-7,05 (4H, m), 7,59-7,66 (4H, m), 8,10 (2H, d, J 4,0).

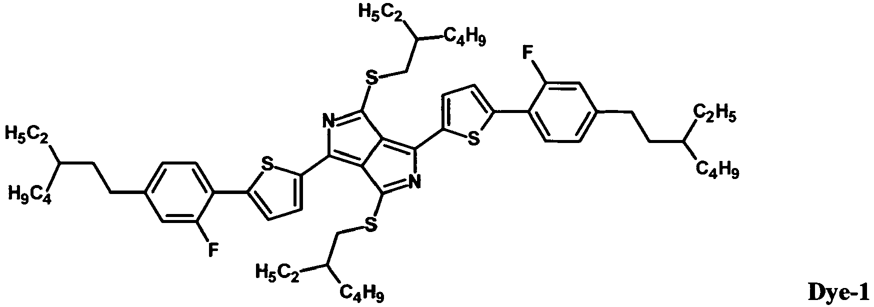

### Grundmischung

Vor- und nachstehend bedeuten
- Δn: die optische Anisotropie gemessen bei 20 °C und 589 nm,
- nₑ: außerordentlicher Brechungsindex gemessen bei 20°C und 589 nm,
- nₒ: ordentlicher Brechungsindex gemessen bei 20°C und 589 nm,
- Δε: die dielektrische Anisotropie bei 20 °C und 1 kHz,
- Kp., T(N,I): Klärpunkt [°C].

| **Phys. Eigenschaften** | **Substanz** | **Massenprozente %** |
|---|---|---|
| Kp.: 114,5°C | CPG-3-F | 5 |
| | CPG-5-F | 5 |
| Δn: 0.1349 | CPU-3-F | 15 |
| nₑ: 1.6303 | CPU-5-F | 15 |
| nₒ: 1.4954 | CP-3-N | 16 |
| | CP-5-N | 16 |
| Δε: 11.3 | CCGU-3-F | 7 |
| | CGPC-3-3 | 4 |
| | CGPC-5-3 | 4 |
| | CGPC-5-5 | 4 |
| | CCZPC-3-3 | 3 |
| | CCZPC-3-4 | 3 |
| | CCZPC-3-5 | 3 |

### Mischungsbeispiel-1:

99.75% Grundmischung + 0.25% Dye-1
Der Farbstoff zeigt bei 664 nm einen Anisotropiegrad von 0.64
Die Extinktionskoeffizienten betragen:

| Wellenlänge | | 1 /[% ^{∗} cm] |
|---|---|---|
| 664 nm | ε_parallel | 1527 |
| 664 nm | ε_senkrecht | 238 |
| 479nm | ε_parallel | 500 |
| 479nm | ε_senkrecht | 254 |

### Anwendungsbeispiel-1:

Eine Mischung folgender Zusammensetzung wird hergestellt:
99,15 % Grundmischung,
0.251% Dye-1
0.165% Dye-2
0.236% Dye-3
0.198% Dye-4

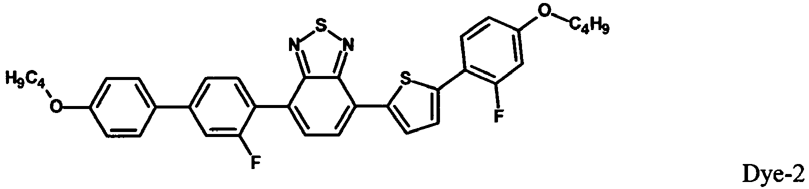

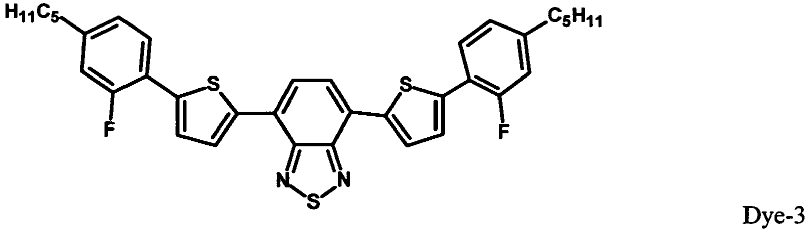

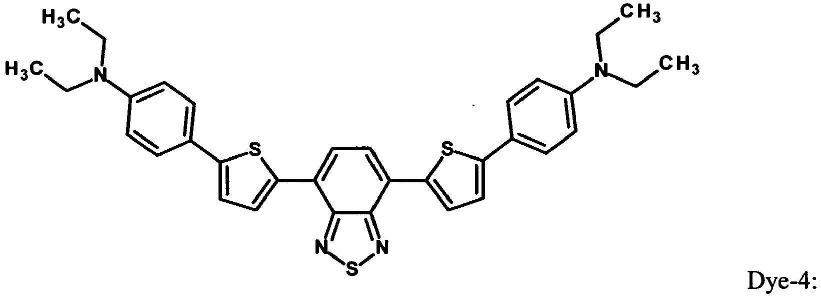

Die Mischung zeigt in einer 25 Mikrometer dicken 90° verdrillten TN-Zelle im Dunkelzustand einen Lichttransmissionsgrad gemäß EN410 von 40% und im Hellzustand einen Lichttransmissionsgrad gemäß EN410 von 71,5%.

Die Farbkoordinaten des Dunkelzustands sind exakt Neutralgrau: x=0.3127 und y = 0.3290 (CIE 1931) und Normlichtart D65
Die Farbkoordinaten des Hellzustands sind : x=0.3167 und y = 0.3379 (CIE 1931) und Normlichtart D65

### Anwendungsbeispiel-2:

Eine Mischung der folgenden Zusammensetzung wird hergestellt:
99,588 % Grundmischung,
0.178% Dye-1
0.048% Dye-2
0.162% Dye-3
0.024% Dye-4

Die Mischung zeigt in einem Aufbau aus zwei 25 Mikrometer dicken 90° verdrillten TN-Zellen in Serie im Dunkelzustand einen Lichttransmissionsgrad gemäß EN410 von 30% und im Hellzustand einen Lichttransmissionsgrad gemäß EN410 von 73%.

Die Farbkoordinaten des Dunkelzustands sind exakt Neutralgrau: x=0.3127 und y = 0.3290 (CIE 1931) und Normlichtart D65
Die Farbkoordinaten des Hellzustands sind : x=0.3207 und y = 0.3472 (CIE 1931) und Normlichtart D65.

## Patentansprüche

1. Verwendung mindestens eines Dithiopyrrolopyrrol-Derivats der Formel (1) 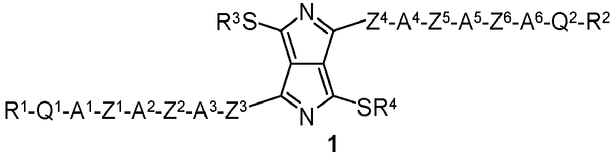 in einer flüssigkristallinen Mischung, worin in der Formel (1)
R¹, R², R³ und R⁴ unabhängig voneinander für lineare oder verzweigte, gegebenenfalls halogensubstituierte Alkylgruppen mit 1 bis 20 Kohlenstoffatomen oder Halogen stehen;
Q¹ und Q² jeweils Einfachbindungen bedeuten oder unabhängig voneinander für O, OCF₂, CF₂, CF₂CF₂, CO stehen;
A¹, A², A³ und A⁴ jeweils Einfachbindungen bedeuten oder unabhängig voneinander für eine Gruppe stehen, die gebildet wird von Phenylen, Pyridin, Pyrimidin, Pyridazin, Naphthylen, Thiophen, Selenophen und Thiazol, mit der Maßgabe, dass
(i) wenigstens A³ und A⁴ aromatische Ringstrukturen darstellen müssen und
(ii) die aromatischen Ringstrukturen gegebenenfalls durch Halogenid substituiert sind; und
Z¹, Z², Z³, Z⁴, Z⁵ und Z⁶ jeweils Einfachbindungen bedeuten oder unabhängig voneinander für ein Radikal stehen, das ausgewählt ist aus der Gruppe, die gebildet wird von C≡C, CH=CH, CF=CF, CF=CH, CH₂CH₂, CF₂-CF₂ und CF₂O, mit der Maßgabe, dass
(iii) wenigstens Z³ und Z⁴ entweder Einfachbindungen oder eine π-konjugierte Gruppe darstellen.

2. Verwendung nach Anspruch 1, wobei in Formel (1) R¹, R², R³ und R⁴ unabhängig voneinander für verzweigte Alkylreste mit 6 bis 12 Kohlenstoffatomen stehen.

3. Verwendung nach Anspruch 1 oder 2, wobei das mindestens eine Derivat der Formel (1) 3, 4 oder 5 aromatische Ringstrukturen enthält.

4. Verwendung nach einem oder mehreren der Ansprüche 1 bis 3, wobei die aromatischen Ringstrukturen in dem mindestens einen Derivat der Formel (1) mit Fluorid substituiert sind.

5. Verwendung nach Anspruch 1, wobei das mindestens eine Derivat der Formel (1) ausgewählt ist aus der Gruppe der Verbindungen 2, 3, 4, 6 und 7: 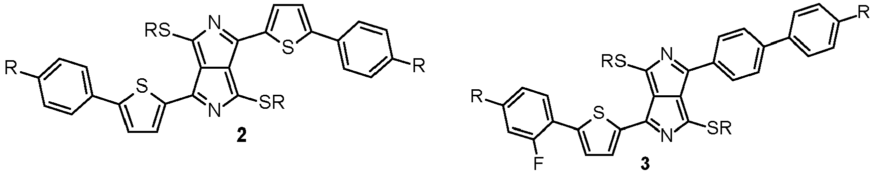 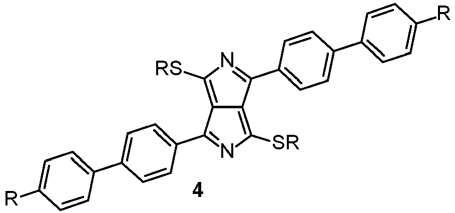 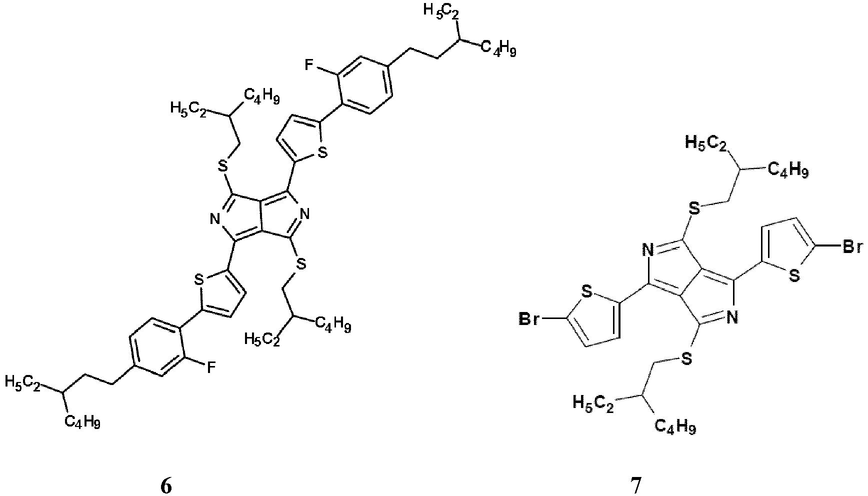 mit der Maßgabe, dass die Reste R die oben angegebene Bedeutung besitzen.

6. Mischung, enthaltend
(a) das mindestens eine Dithiopyrrolopyrrol-Derivat der Formel (1) gemäß einem oder mehreren der Ansprüche 1 bis 5 sowie
(b1) mindestens eine flüssigkristalline Verbindung.

7. Mischung nach Anspruch 6, ferner enthaltend
(b2) mindestens einen chiralen Dotierstoff und/oder
(b3) mindestens einen Stabilisator und/oder
(b4) mindestens einen vorzugsweise ebenfalls dichroitischen Farbstoff, der von denen der Formel (1) verschieden ist.

8. Verwendung der Mischung nach Anspruch 6 oder 7 in einer Vorrichtung zur Regulierung des Lichteintritts in einen Raum.

9. Vorrichtung zur Regulierung des Lichteintritts in einen Raum, enthaltend die Mischung nach Anspruch 6 oder 7.

10. Vorrichtung nach Anspruch 9, **dadurch gekennzeichnet, dass** sie folgende Schichtabfolge aufweist, wobei zusätzlich weitere Schichten vorhanden sein können:
• Substratschicht;
• Elektrisch leitfähige transparente Schicht;
• Orientierungsschicht;
• Schaltschicht enthaltend die Mischung nach Anspruch 6 oder 7;
• Orientierungsschicht;
• Elektrisch leitfähige transparente Schicht;
• Substratschicht.

11. Fenster, enthaltend eine Vorrichtung nach Anspruch 9 oder 10.

## Claims

1. Use of at least one dithiopyrrolopyrrole derivative of the formula (1) 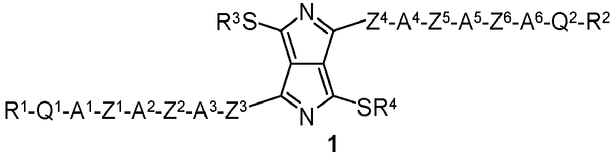 in a liquid-crystalline mixture, in which, in the formula (1),
R¹, R², R³ and R⁴, independently of one another, stand for linear or branched, optionally halogen-substituted alkyl groups having 1 to 20 carbon atoms or halogen;
Q¹ and Q² each denote single bonds or stand, independently of one another, for O, OCF₂, CF₂, CF₂CF₂ or CO;
A¹, A², A³ and A⁴ each denote single bonds or stand, independently of one another, for a group formed by phenylene, pyridine, pyrimidine, pyridazine, naphthylene, thiophene, selenophene and thiazole, with the proviso that
(i) at least A³ and A⁴ must represent aromatic ring structures and
(ii) the aromatic ring structures are optionally substituted by halide; and
Z¹, Z², Z³ Z⁴, Z⁵ and Z⁶ each denote single bonds or stand, independently of one another, for a radical selected from the group formed by C≡C, CH=CH, CF=CF, CF=CH, CH₂CH₂, CF₂-CF₂ and CF₂O, with the proviso that
(iii) at least Z³ and Z⁴ represent either single bonds or a π-conjugated group.

2. Use according to Claim 1, where R¹, R², R³ and R⁴ in formula (1) stand, independently of one another, for branched alkyl radicals having 6 to 12 carbon atoms.

3. Use according to Claims 1 or 2, where the at least one derivative of the formula (1) contains 3, 4 or 5 aromatic ring structures.

4. Use according to one or more of Claims 1 to 3, where the aromatic ring structures in the at least one derivative of the formula (1) are substituted by fluoride.

5. Use according to Claim 1, where the at least one derivative of the formula (1) is selected from the group of compounds 2, 3, 4, 6 and 7: 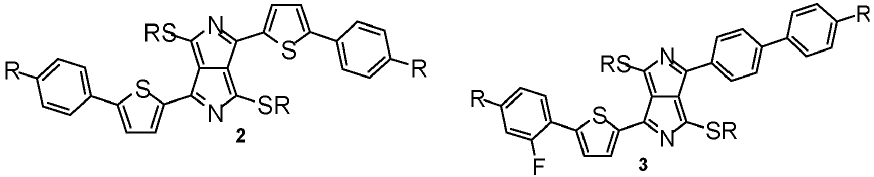 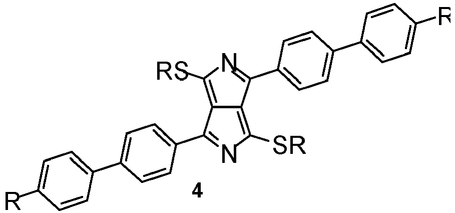 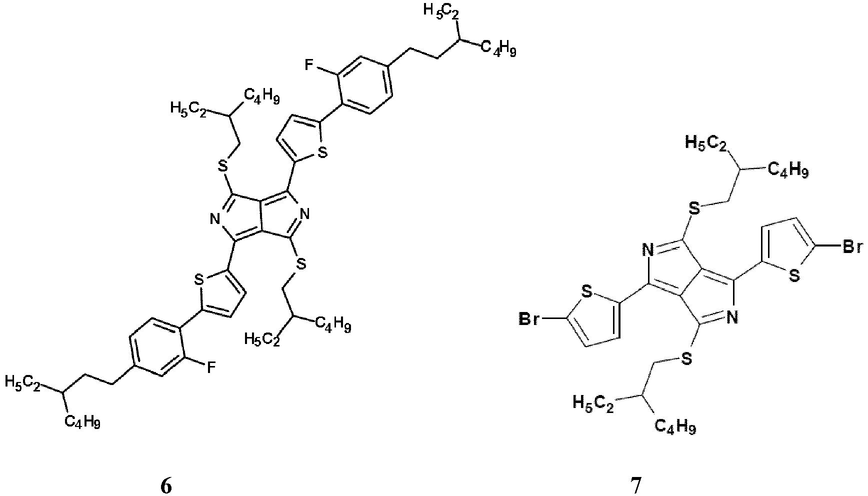 with the proviso that the radicals R have the meaning indicated above.

6. Mixture comprising
(a) the at least one dithiopyrrolopyrrole derivative of the formula (1) according to one or more of Claims 1 to 5 and
(b1) at least one liquid-crystalline compound.

7. Mixture according to Claim 6, furthermore comprising
(b2) at least one chiral dopant and/or
(b3) at least one stabiliser and/or
(b4) at least one dye, preferably likewise dichroic, which is different from those of the formula (1).

8. Use of the mixture according to Claim 6 or 7 in a device for regulating the entry of light into a room.

9. Device for regulating the entry of light into a room, containing the mixture according to Claim 6 or 7.

10. Device according to Claim 9, **characterised in that** it has the following layer sequence, where further layers may additionally be present:
• substrate layer;
• electrically conductive transparent layer;
• alignment layer;
• switching layer comprising the the mixture according to Claim 6 or 7;
• alignment layer;
• electrically conductive transparent layer;
• substrate layer.

11. Window containing a device according to Claim 9 or 10.

## Revendications

1. Utilisation d'au moins un dérivé de dithiopyrrolopyrrole de formule (1) 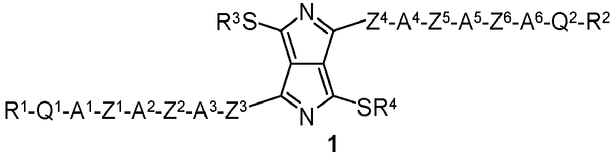 dans un mélange liquide-cristallin,
dans lequel, dans la formule (1),
R¹, R², R³ et R⁴, indépendamment les uns des autres, représentent des groupements alkyle linéaires ou ramifiés, éventuellement à substitution halogène, ayant de 1 à 20 atomes de carbone ou halogène ;
Q¹ et Q² désignent chacun des liaisons simples ou représentent, indépendamment l'un de l'autre, O, OCF₂, CF₂, CF₂CF₂ ou CO ;
A¹, A², A³ et A⁴ désignent chacun des liaisons simples ou représentent, indépendamment les uns des autres, un groupement formé par phénylène, pyridine, pyrimidine, pyridazine, naphtylène, thiophène, sélénophène et thiazole, à condition que
(i) au moins A³ et A⁴ doivent représenter des structures de cycles aromatiques et
(ii) les structures de cycles aromatiques sont éventuellement substituées par halogénure ; et
Z¹, Z², Z³, Z⁴, Z⁵ et Z⁶ désignent chacun des liaisons simples ou représentent, indépendamment les uns des autres, un radical choisi dans le groupe constitué par C≡C, CH=CH, CF=CF, CF=CH, CH₂CH₂, CF₂-CF₂ et CF₂O, à condition que
(iii) au moins Z³ et Z⁴ représentent des liaisons simples ou bien un groupement π-conjugué.

2. Utilisation selon la revendication 1, dans laquelle R¹, R², R³ et R⁴ dans la formule (1) représentent, indépendamment les uns des autres, des radicaux alkyle ramifiés ayant de 6 à 12 atomes de carbone.

3. Utilisation selon les revendications 1 ou 2, dans laquelle le au moins un dérivé de formule (1) contient 3, 4 ou 5 structures de cycles aromatiques.

4. Utilisation selon l'une ou plusieurs parmi les revendications 1 à 3, dans laquelle les structures de cycles aromatiques dans le au moins un dérivé de formule (1) sont substituées par fluorure.

5. Utilisation selon la revendication 1, dans laquelle le au moins un dérivé de formule (1) est choisi dans le groupe constitué par les composés 2, 3, 4, 6 et 7 : 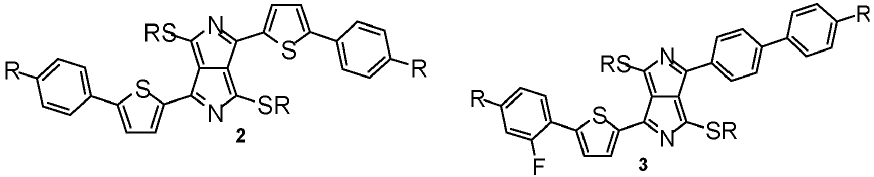 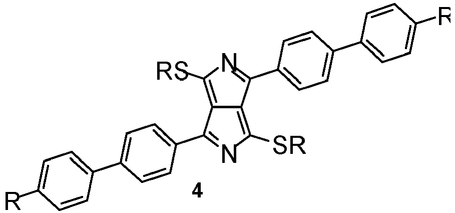 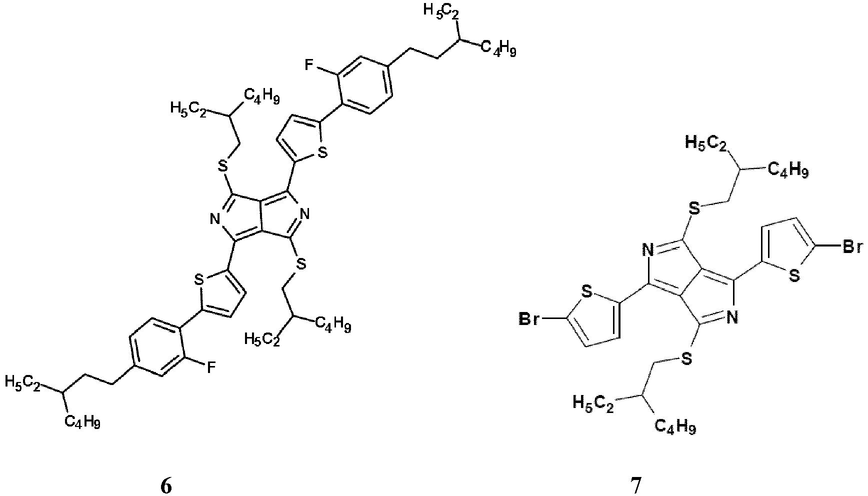 à condition que les radicaux R revêtent la signification indiquée ci-dessus.

6. Mélange comprenant
(a) le au moins un dérivé de dithiopyrrolopyrrole de formule (1) selon l'une ou plusieurs parmi les revendications 1 à 5 et
(b1) au moins un composé liquide-cristallin.

7. Mélange selon la revendication 6, comprenant en outre
(b2) au moins un dopant chiral et/ou
(b3) au moins un stabilisant et/ou
(b4) au moins un colorant, de même de préférence dichroïque, qui est diffèrent de ceux de formule (1).

8. Utilisation du mélange selon la revendication 6 ou 7 dans un dispositif destiné à réguler l'entrée de la lumière dans une pièce.

9. Dispositif destiné à réguler l'entrée de la lumière dans une pièce, contenant le mélange selon la revendication 6 ou 7.

10. Dispositif selon la revendication 9, **caractérisé en ce qu'il** possède la séquence de couches suivante, où d'autres couches peuvent en outre être présentes :
• une couche de substrat ;
• une couche transparente électriquement conductrice ;
• une couche d'alignement ;
• une couche de commutation comprenant le mélange selon la revendication 6 ou 7;
• une couche d'alignement ;
• une couche transparente électriquement conductrice ;
• une couche de substrat.

11. Fenêtre contenant un dispositif selon la revendication 9 ou 10.
